# EUROPEAN PATENT APPLICATION

(11) **EP 2 078 491 A1**
(43) Date of publication of application: **15.07.2009**
(21) Application number: 08021817.5
(22) Date of filing: 16.12.2008
(51) Int. Cl.: A61B 1/018, A61B 17/06

(54) **T-Bar suture instrument**

(30) Priority: 14.01.2008 US 13771
(71) Applicant: Olympus Medical Systems Corporation, Tokyo 151-0072 (JP)
(72) Inventor: Shiono, Junji, Tokyo 151-0072 (JP); Hayashi, Kensuke, Tokyo 151-0072 (JP); Muyari, Yuta, Tokyo 151-0072 (JP); Suzuki, Takayuki, Tokyo 151-0072 (JP)
(74) Representative: von Hellfeld, Axel

(57) **Abstract**

An endoscope treatment instrument having a needle for puncturing a biological tissue in a body cavity includes an indicator region provided on the outer surface of the needle including an indicator shape having miniature concave portions, and a plain region without the indicator shape provided in connection with at least one side of the indicator region in the axial line direction of the needle.

## Description

### BACKGROUND OF THE INVENTION

### Field of the Invention

The present invention relates to an endoscope treatment tool that is used by inserting into a body cavity.

### Description of the Related Art

An endoscope treatment tool that uses suture thread and which is provided with anchors on both edges for the purpose of suturing a perforation or a laceration formed in a hollow organ such as the stomach or the intestines or the like is conventionally known (see International Patent Application Publication No. WO 2007/037326). With this suture instrument, the suture thread is anchored to a biological tissue by discharging the anchors of the suture thread after loading the suture thread into a cavity of a hollow needle, puncturing or penetrating the needle into a biological tissue around the perforation or the like.

In most cases, the biological tissue to be treated with this kind of endoscope treatment instrument is located close to other organs or biological tissues. Therefore, in order to perform procedures safely, the operator needs to confirm precisely the puncturing amount of the needle.

As a method to confirm the puncturing amount, a method such as fixing a protruding length of the needle to a predetermined length in advance is known for needles used for local injections or the like. However, since the above-mentioned endoscope treatment instrument is used for various regions, when such a method is employed, the versatility of the treatment instrument decreases.

Also, since the endoscope treatment instrument usually has a long insertion portion, when the endoscope zigzagged in the body cavity for example, the protruding length set by an operation portion sometimes differs from the actual protruding length of the needle. Accordingly, the above-mentioned method is not always precise.

### SUMMARY OF THE INVENTION

The present invention was achieved in view of the above circumstances, and has as its main object the provision of an endoscope treatment instrument that can easily confirm a puncturing amount.

A first aspect of the present invention is an endoscope treatment instrument having a needle which is punctured into a biological tissue in a body cavity, including: an indicator region provided on the outer surface of the needle and including an indicator shape having miniature concave portions, a plain region without the indicator shape provided in connection with at least one side of the indicator region in the axial line direction of the needle.

A second aspect of the present invention is puncturing method into a biological tissue in a body cavity using the endoscope treatment instrument of the present invention including determining the puncturing amount of the needle by referring the indicator region of the needle by an endoscopic image.

### BRIEF DESCRIPTION OF THE DRAWINGS

FIG 1 shows a state in which a suture instrument, which is an endoscope treatment instrument in accordance with a first embodiment of the present invention, is used.
FIG 2 shows a suture instrument in accordance with the first embodiment of the present invention.
FIG. 3 shows an enlarged cross-sectional view of a distal end portion of the suture instrument.
FIG. 4 shows a suture unit that is used for the suture instrument.
FIG. 5 is a perspective view that shows a needle of the suture instrument and the suture unit.
FIG. 6A is a lateral view of the needle and FIG. 6B is a development view of the needle.
FIG. 7 is a cross-sectional view of an operation portion of the suture instrument.
FIG. 8 shows an operation when the suture instrument is used.
FIG. 9 shows when a .mucous membrane of a stomach is resected by using a resecting instrunment.
FIG 10A is an enlarged cross-sectional view of the operation portion when the suture instrument is used and FIG. 10B is an enlarged cross-sectional view of the distal end portion when the suture instrument is used.
FIG. 11 shows an operation of the distal end of the endoscope device to which the suture instrument is inserted during use.
FIG. 12 shows an operation of the distal end of the endoscope device to which the suture instrument is inserted during use.
FIG. 13 is a view which shows a state where a biological tissue around the region where the mucous membrane is resected is grasped by a grasping forceps.
FIG 14 shows an endoscopic image when the biological tissue is grasped by the grasping forceps.
FIG 15A is an enlarged cross-sectional view of a distal end operation portion of the suture instrument during use and FIG. 15B is an enlarged cross-sectional view of the distal end portion of the suture instrument during use.
FIG. 16 shows a state when a first anchor is discharged.
FIG. 17 shows an endoscopic image when the first anchor is discharged.
FIG. 18A is an enlarged cross-sectional view of the distal end operation portion of the suture instrument during use and FIG. 18B is an enlarged cross-sectional view of the distal end portion of the suture instrument during use.
FIG. 19 shows a state when a second anchor is discharged.
FIG. 20 shows a state when a second anchor is discharged.
FIG. 21 shows a process of suturing by the suture instrument.
FIG. 22 shows a state in which the suturing by the suture instrument is completed.
FIG. 23A to FIG. 23C show a needle of alternative examples of the suture instrument.
FIG. 24A shows a puncturing by a local injection syringe into a biological tissue around the region where the mucous membrane is resected.
FIG. 24B shows a state in which a saline solution is locally injected by the local injection syringe.
FIG. 25 shows an endoscopic image when the suture instrument is used.
FIG. 26 shows a state when a first anchor is discharged.
FIG. 27 shows a state when a second anchor is discharged.
FIG. 28 shows a state when a second anchor is discharged.
FIG. 29 shows a state in which the suturing by the suture instrument is completed.
FIG. 30 shows a state in which a local injection syringe, which is an endoscope treatment instrument in accordance with a second embodiment of the present invention, is used.
FIG 31 shows an endoscopic image when the local injection syringe is used.
FIG. 32 shows an image taken by ultrasound tomography when the local injection syringe is used.

### DETAILED DESCRIPTION OF THE INVENTION

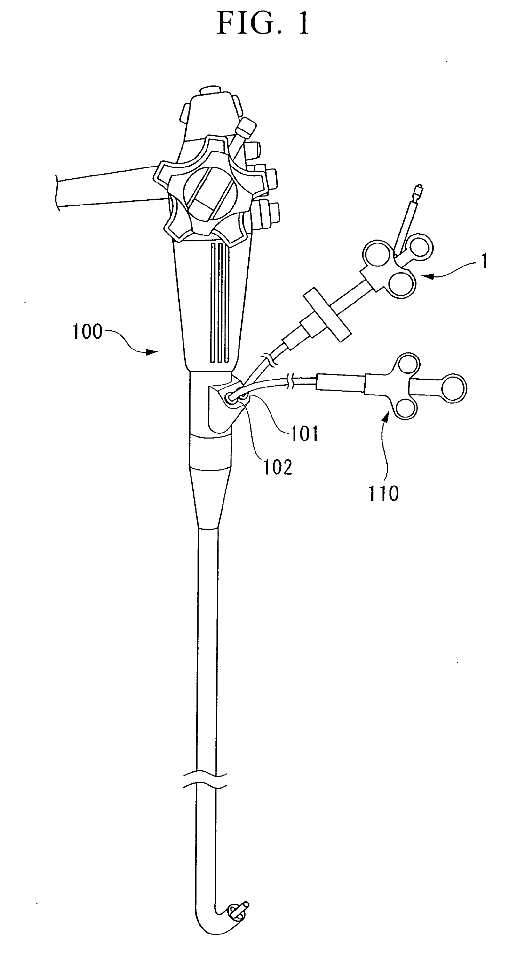

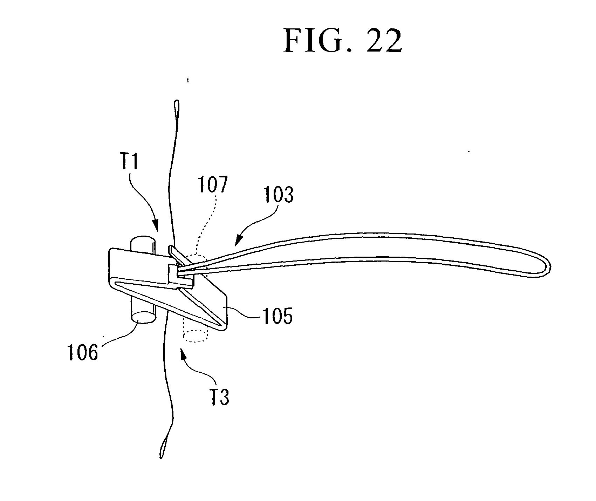

An endoscope treatment instrument in accordance with a first embodiment of the present invention shall be described with reference to FIG. 1 to FIG. 29.

FIG. 1 shows a state in which a suture instrument 1, which is an endoscope treatment instrument in accordance with the present embodiment, is used. An endoscope device 100, into which the suture instrument 1 is inserted, is a two-channel endoscope in which the suture instrument 1 is inserted into a first channel 101. In FIG. 1, a grasping forceps 110 is inserted into a second channel 102. But other treatment instruments such as a snare wire or the like is arbitrarily inserted by being replaced.

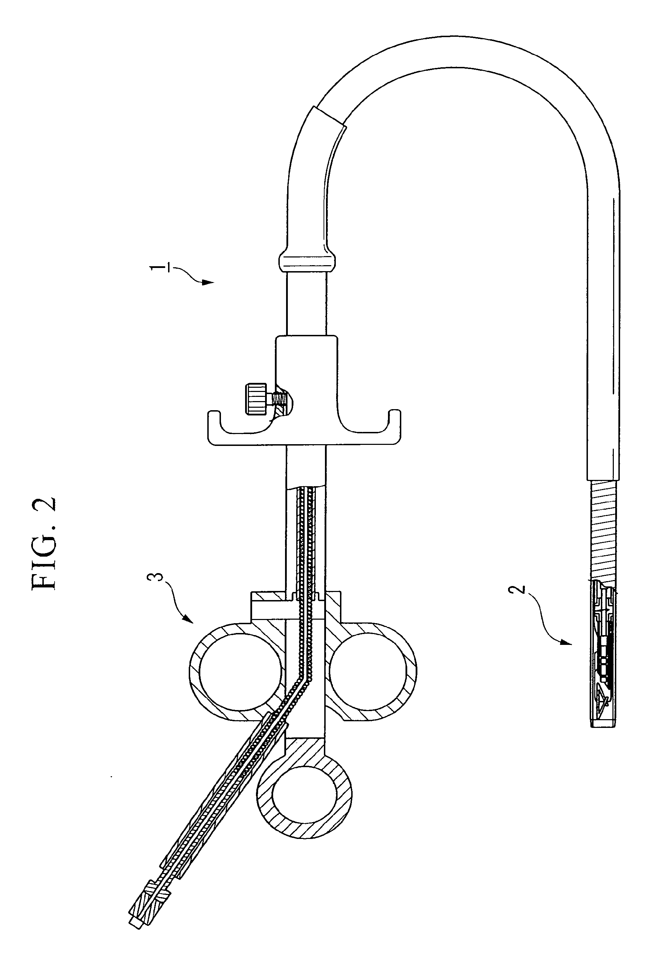

FIG. 2 is a partial cross-sectional view showing the suture instrument 1. The suture instrument 1 is provided with a distal end portion 2 which is inserted into a body and an operation portion 3 for operating respective mechanisms of the distal end portion 2.

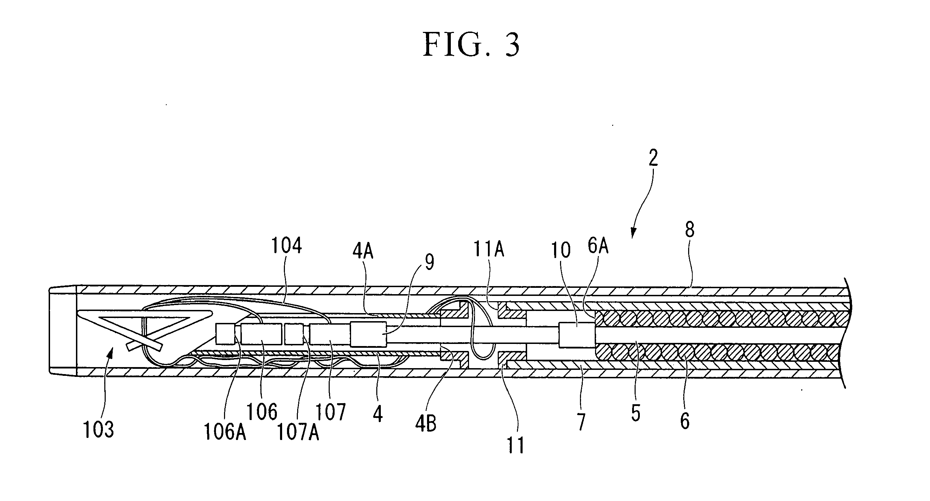

FIG. 3 shows an enlarged cross-sectional view of the distal end portion 2. The distal end portion 2 is provided with: a needle 4 to which a suture unit which is to be described later is attached, a wire 5 which is inserted in the needle 4, a second sheath 6 in which a proximal end of the wire 5 is inserted, a tube 7 which is integrally fixed to the needle 4 at the side of the proximal end of the needle 4 in which the second sheath 6 and the wire 5 are inserted so as to be able to extend and retract in the axial line direction, and a first sheath 8 in which the tube 7 is inserted.

The needle 4 is a hollow member made of metal or the like in which a groove 4A is formed on the upper surface. An anchor of the suture unit is housed inside of the needle 4.

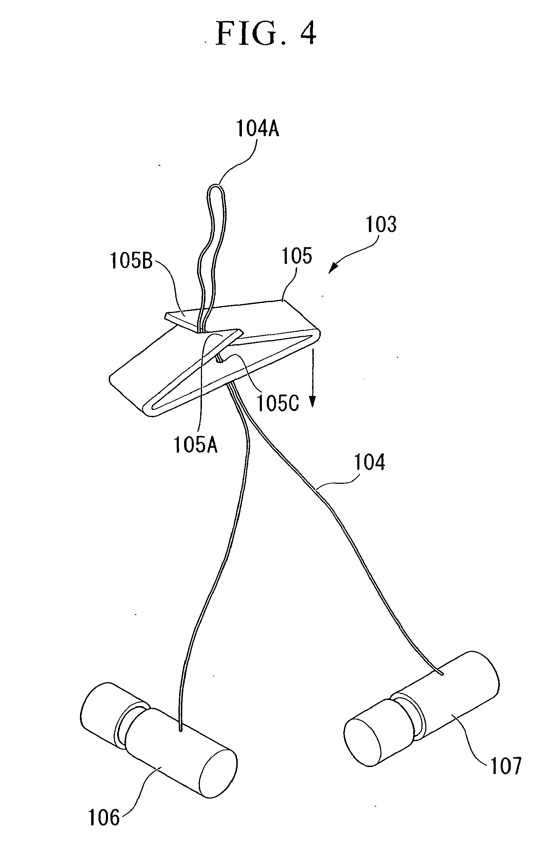

FIG. 4 shows a suture unit 103 that is housed in the needle 4. The suture unit 103 is provided with a suture thread 104, stopper 105 through which the suture thread 104 is passed, and a rod-shaped first anchor 106 and a second anchor 107 attached to both ends of the suture thread 104.

The stopper 105 is formed such that end portions of a right side 105A and a left side 105B, which are plate-shaped members made of metal, resin such as biodegradable resin or the like for example, are folded back so as to face each other and the end portions 105A and 105B are engaged with each other.

A hole 105C is provided substantially in the center of the stopper 105 in the longitudinal direction of the stopper 105. The suture thread 104 which is folded back at a folding portion 104A which is located in an arbitral position in the middle thereof is passed through the hole 105C from the opposite side of the end portions 105A and 105B, and is disposed so as to pass through between the end portions 105A and 105B which are engaged with each other. The operation of the stopper 105 when it is used will be described later.

As shown in FIG. 3, inside of the needle 4, the first anchor 106 and the second anchor 107 of the suture unit 103 are housed to be aligned in the axial line direction with the first anchor 106 being disposed at the side of the distal end. The suture thread 104 connected to each of the anchors 106 and 107 is exposed to the outside of the needle 4 from the groove 4A.

On the outer surface of each of the anchors 106 and 107, engaging grooves 106A and 107A are provided over a whole circumference respectively. Each of the engaging grooves 106A and 107A is engaged with engaging protrusions (not shown) which are respectively provided on the side of the inner hollow of the needle 4. The engaging protrusions prevent each of the anchors from discharging by mistake or falling by their own weight when the distal end of the needle 4 is directed perpendicularly downward.

The wire 5 is made of metal or the like and the distal end thereof is insertred through the needle 4 from the proximal end 4B of the needle 4. On the distal end of the wire 5, a pushing member 9 is attached. By extending the wire 5 along the axial line direction to the side of the distal end of the needle 4, it is possible to push the first anchor 106 and the second anchor 107 and discharge them outside of the needle 4.

It is preferable that the wire 5 be a single wire which is able to preferably propagate the pushing force added at the operation portion 3 to the pushing member 9, but a multiwire which is made by twisting metal wires, a coil wire which is made of the metal wire or the multiwire twisted in a coil-shape, and the like can also be used.

An annular abutting member 10 which maintains a relative position between the wire 5 and the second sheath 6 constant is attached and fixed to the wire 5 in a position away from the pushing member 9 by a predetermined distance. The outer diameter of the abutting member 10 is set so as to be able to freely extend and retract inside of the needle 4. Note that the abutting member 10 may protrude outward in the radial direction from the wire 5 and does not necessarily need to be an annular shape.

The second sheath 6 is a coil sheath which is formed such that a metal wire or a multiwire is twisted in a cylindrical shape. The proximal end of the wire 5 is inserted in the second sheath 6 in the axial line direction so as to be able to extend and retract. The inner diameter of the second sheath 6 is set smaller than the outer diameter of the abutting member 10 of the wire 5 so that the abutting member 10 abuts the distal end 6A of the second sheath 6, whereby the abutting member 10 is not able to be inserted into the second sheath 6. That is, when the abutting member 10 and the distal end 6A of the second sheath 6 abut, a relative position between the wire 5 and the second sheath 6 is kept constant.

The tube 7 is a cylindrical member having flexibility made of resin or the like. As a material of the tube 7, a resin member or the like which does not extend much in the axial line direction is preferable. The tube 7 is connected integrally to the proximal end 4B of the needle 4 via a connection pipe 11 which is attached to the distal end of the tube 7.

On the outer surface of the connection pipe 11, a through-hole 11A which penetrates the inner cavity is provided. The folding portion 104A of the suture thread 104 of the suture unit 103 is inserted in the inner cavity of the connection pipe 11 from the through-hole 11A and the wire 5 which is inserted in the inner portion passes through a loop formed by the folding portion 104A.

The inner diameter of the connection pipe 11 in the axial line direction is set greater than the outer diameter of the abutting member 10 of the wire 5. The abutting member 10 is able to freely extend and retract in the connection pipe 11 along the axial line direction. On the other hand, the inner diameter of the connection pipe 11 in the axial line direction is set smaller than the outer diameter of the second sheath 6 so that the second sheath 6 is not able to pass into the connection pipe 11.

The first sheath 8 is a coil sheath having the same constitution as the second sheath 6. The tube 7 and the needle 4, which is integrally connected to the tube 7, are inserted in the first sheath 8 to be able to extend and retract in the axial line direction. As shown in FIG. 3, it is possible to house the entire suture unit 103, which is placed in the needle 4, in the inner cavity of the first sheath 8.

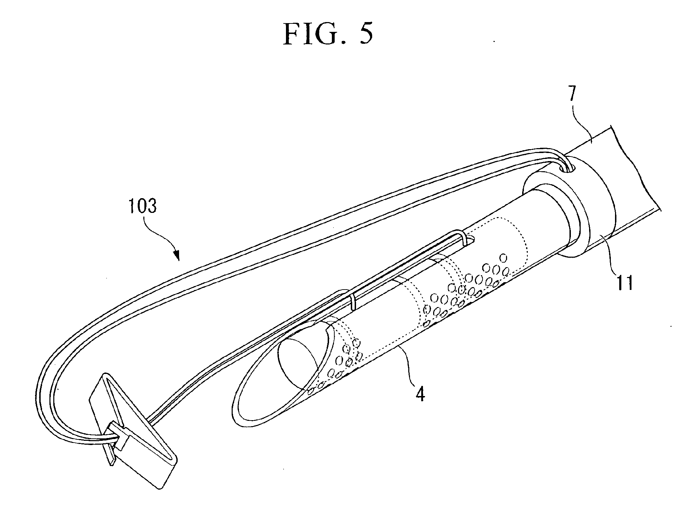

FIG. 5 is a perspective view that shows the needle 4. On the outer surface of the needle 4, a plurality of dimples 12 for confirming the puncturing amount of the needle 4 is provided.

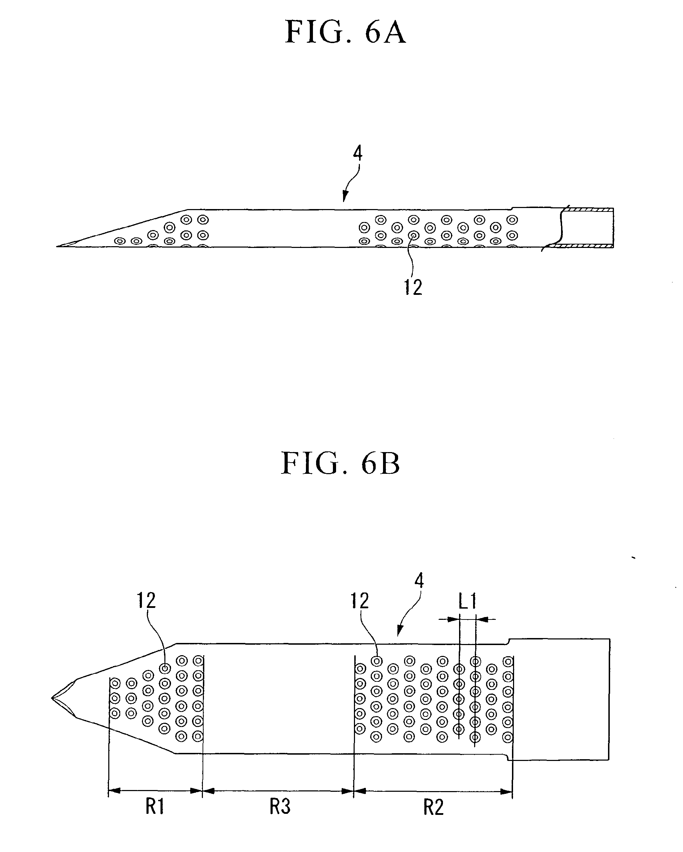

FIG. 6A is a lateral view of the needle 4 and FIG. 6B is a development view of the needle 4. The diameter of each of the dimples 12 is about 0.3 mm. The dimples 12 are provided on the outer surface of the needle 4 by laser or the like so that they become miniature concave portions with a substantially spherical shape. The depth of the dimples 12 is set so that they do not penetrate the needle 4 and communicate with the inner cavity of the needle 4.

As shown in FIG. 6B, the dimples 12 form a plurality of lines along the circumferential direction of the needle 4. On the outer surface of the needle 4, a first indicator region R1 at the side of the distal end where six lines of the dimples 12 are disposed, and a second indicator region R2 where ten lines of the dimples 12 are disposed are formed. A gap between the proximal end of the first indicator region R1 and the distal end of the second indicator region R2 is about 5 mm and a plain region R3, in which the dimples are not formed, is formed in the gap.

The length from the distal end of the needle 4 to the proximal end of the first indicator region R1 is set to be about 5 mm. The length from the distal end of the second indicator region R2 to the proximal end thereof is set to be about 5 mm. In the first indicator region R1 and the second indicator region R2, the length L1 between the adjacent lines of dimples is set to be about 0.5 mm.

Accordingly, due to the process performed on the outer surface of the needle 4 so as to form the proximal end of the first indicator region R1, the distal end and the proximal ends of the second indicator region R2 indicators, it is possible to confirm the puncturing amount of the needle 4 every 5 mm. Furthermore, in the first indicator region R1 and the second indicator region R2, by making each of the lines of the dimples 12 indicators, it is possible to confirm the puncturing amount of the needle 4 for every 0.5 mm.

Note that dimensions of each of the indicator regions R1, R2, and the plain region R3 and an interval between the lines of the dimples 12 and the like are not limited to the above-mentioned values, but they can be arbitrarily set.

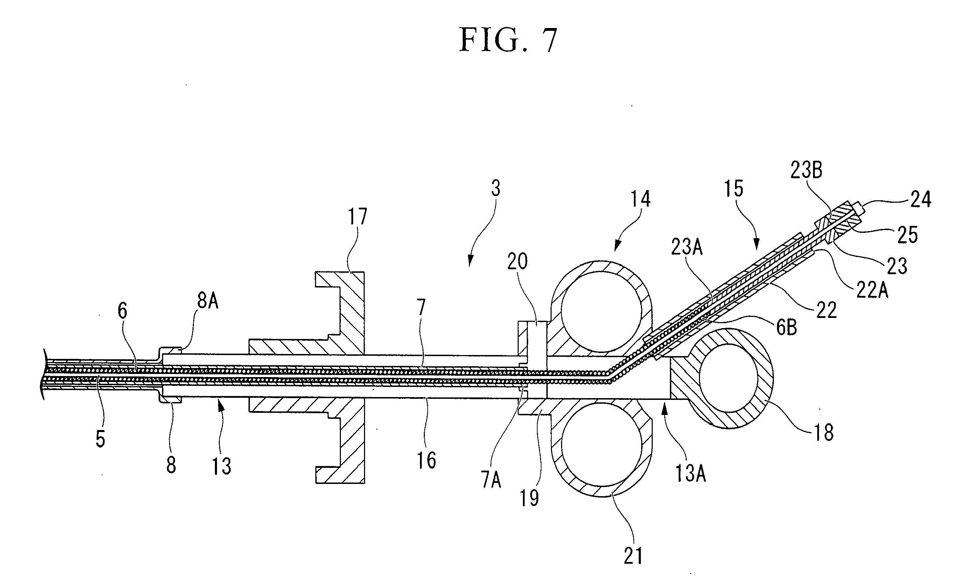

FIG. 7 is a cross-sectional view of an operation portion 3. The operation portion 3, which is provided at the side of the proximal end of the wire 5 and the second sheath 5, includes: a main body 13 to which the proximal end of the first sheath 8 is connected, a sliding portion 14 attached to the main body 13 so as to be able to slide in the axial line direction of the main body 13, and a distal end operation portion 15 fixed to the sliding portion 14.

The main body 13 is made of resin or the like and a pair of sidewall members 16, each of which has a rod shape, is aligned in parallel. In the vicinity of the distal end of the main body 13, a substantially cylindrical-shaped adjuster 17 made of resin or the like is attached so as to surround the pair of sidewall members 16.

The adjuster 17 is able to slide in the axial line direction of the main body 13. The adjuster 17 can be fixed to an arbitrary position relative to the main body 13 by a fixing means (not shown) such as a screw or the like. By changing the fixing position of the adjuster 17, as described later, it is possible to adjust a protruding amount of the needle 4 from the first sheath 8.

At the distal end of the main body 13, a pair of sidewall members 16 is integrated and an annular-shaped holding portion 18 is provided.

The sliding portion 14 is provided with a slider 19 attached to the main body 13 so as to be able to slide relative to the main body 13 and a connection member 20 fixed to the slider 19.

The slider 19 is a substantially cylindrical member made of resin or the like which is attached to the main body 13 at a side closer to the proximal end 13A of the main body than the adjuster 17 so as to surround the pair of sidewalls 16. The slider 19 is able to slide along the axial line direction of the main body 13 between the adjuster 17 and the holding portion 18. A handle 21 is provided in the slider 19 for holding onto during operation.

The connection member 20 is made of resin, metal, or the like. The proximal end 7A of the tube 7, which extends between the pair of sidewall members 16 from the proximal end 8A of the first sheath 8, is fixed to the connection member 20 by way of welding or adhesion or the like. That is, the proximal end 7A of the tube 7 is fixed to the slider 19 via the connection member 20 and so it is possible to make the tube 7 extend and retract by a predetermined range along the axial line direction of the main body 13 by sliding the slider 19.

The distal end operation portion 15 is provided with a cylindrical member 22 fixed to the slider 19, a sheath operation member 23 inserted through the cylindrical member 22, and a wire operation knob 24 attached to the proximal end of the wire 5.

The cylindrical member 22 is made of resin or the like and is fixed to the rear of the handle 21 of the slider 19. The second sheath 6 and the wire 5 extending from the proximal end 7A from the tube 7 are inserted in the cylindrical member 22.

The sheath operation member 23 is a cylindrical member made of resin or the like and is inserted in the cylindrical member 22 from the side of the proximal end 22A of the cylindrical member 22. The sheath operation member 23 is able to slide along the axial line of the cylindrical member 22. The proximal end 6B of the second sheath 6 is connected to the distal end 23A of the sheath operation member 23 by means of adhesion, caulking, or the like. The wire 5 extending from the proximal end 6B of the second sheath 6 is exposed from the proximal end 23B of the sheath operation member 23 by passing through the inner cavity of the sheath operation member 23.

The wire operation knob 24 is a disk-shaped member which is attached to the proximal end of the wire 5 exposed from the proximal end 23B of the sheath operation member 23. The wire operation knob 24 is not limited to a disk-shape, but any shape, such as a rod-shape as the first anchor 106 of the suture unit 103, is available as long as it locks to the sheath operation member 23 and the wire stopper which is described later.

A wire stopper 25 for maintaining the positional relationship between the wire 5 and the second sheath 6 constant and preventing malfunctions is provided between the proximal end 23B of the sheath operation member 23 and the wire operation knob 24 so as to be freely attached and detached.

One example of the wire stopper 25 is a member with a substantially C-shaped cross-section with a portion of the outer circumference cut away from a substantially cylindrical member. However, the wire stopper 25 is not limited to this, but any member, such as a clip which is able to be attached to the wire 5, is available as long as it is possible to maintain the positional relationship between the wire 5 and the second sheath 6 constant. In the suture instrument 1 of the present embodiment, when the wire stopper 25 is attached, the abutting member 10 and the distal end 6A of the second sheath 6 abut.

The operation of the suture instrument 1 during use as constituted above shall be described taking a case in which a mucous membrane of a stomach is resected, as an example.

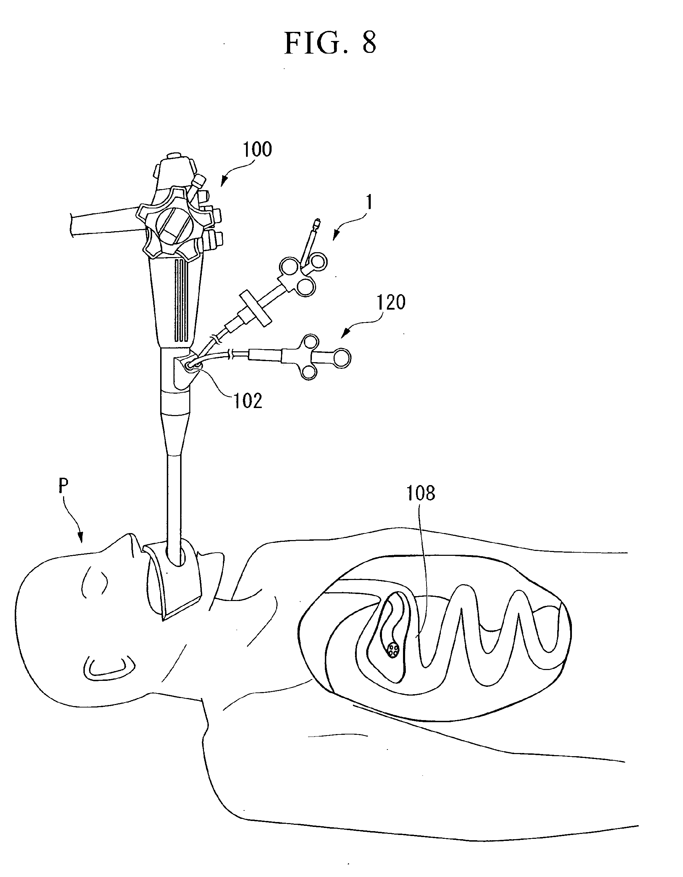

As shown in FIG. 8, an operator penetrates the endoscope device 100 into a body of a patient P and moves the distal end of the endoscope 100 to the vicinity of the biological tissue which is to be treated inside of the stomach 108. Note that at this moment, in the second channel 102, a resecting instrument 120 having a snare wire is inserted.

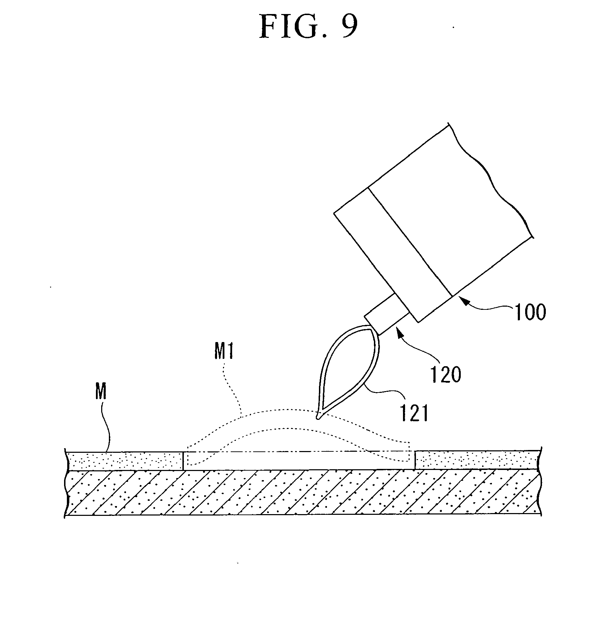

Next, as shown in FIG. 9, the resecting instrument 120 is protruded from the distal end of the endoscope device 100 and a part M1 of a mucous membrane M is resected by a snare wire 121 with a tumor or the like located below.

Next, the mucous membrane M1 is resected and a portion where a submucosal tissue is exposed is sutured by the grasping forceps 110 and the suture instrument 1. The operator withdraws the resecting instrument 120 from the second channel 102 and inserts the grasping forceps 110 into the second channel 102. And the distal end of the first sheath 8 of the suture instrument 1 is protruded from the distal end of the endoscope device 100.

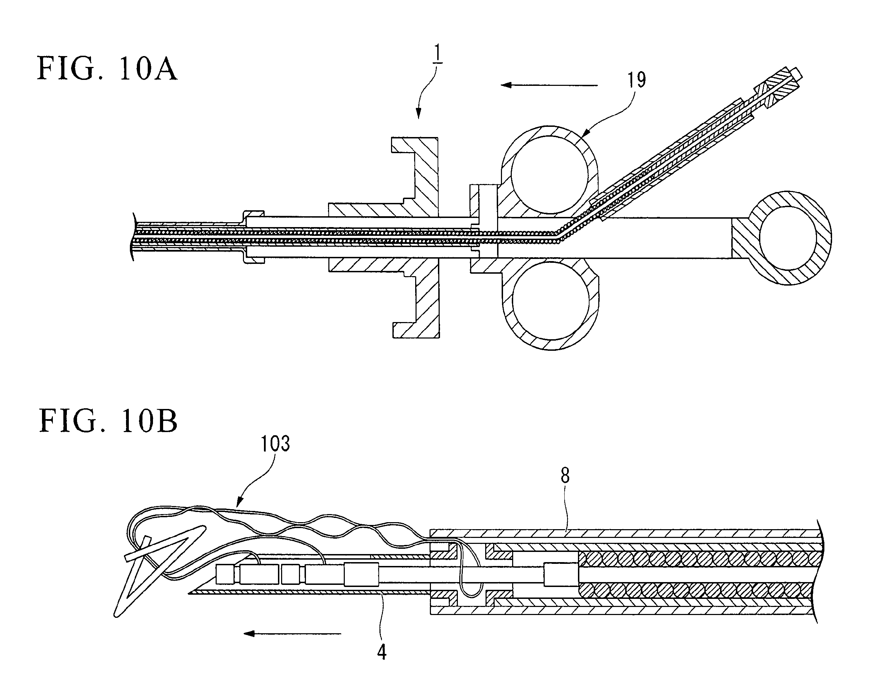

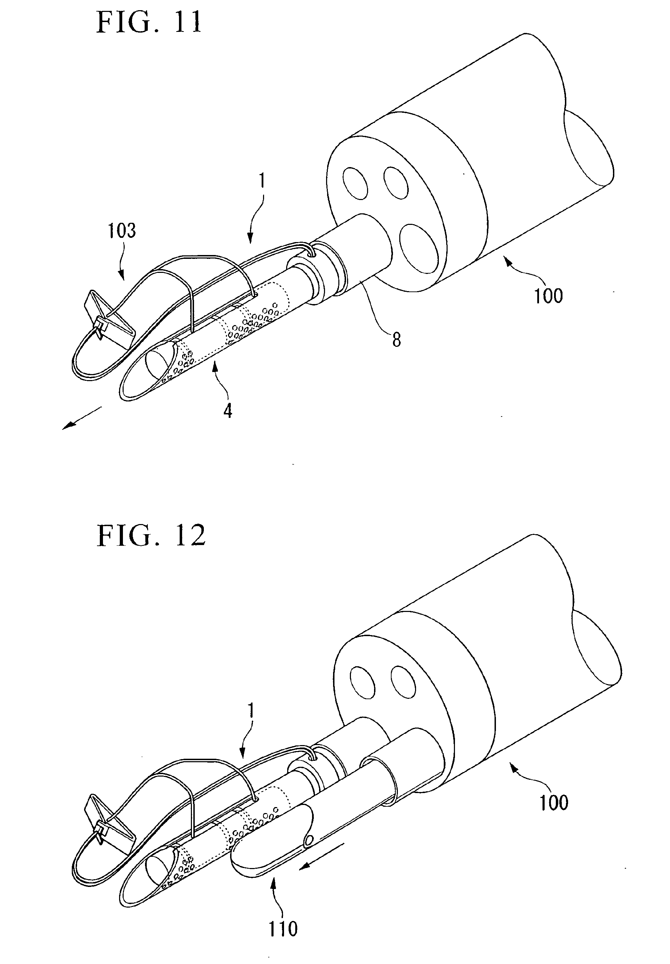

As shown in FIG. 10A, the operator slides the slider 19 forward. Then as shown in FIGS. 10B and 11, the needle 4 and the suture unit 103 attached to the needle 104 are exposed from the distal end of the first sheath 8. At this moment, a fixing position of the adjuster 17 relative to the main body 13 is adjusted if needed and by making the slider 19 abut the adjuster 17, a protruding amount of the needle 4 from the first sheath 8 may be adjusted.

Note that when the slider 19 is slid forward, the distal end operation portion 15 is moved forward accordingly, whereby a relative position between the combination of the tube 7 and needle 4 and the combination of the wire 5 and the second sheath 6 does not change.

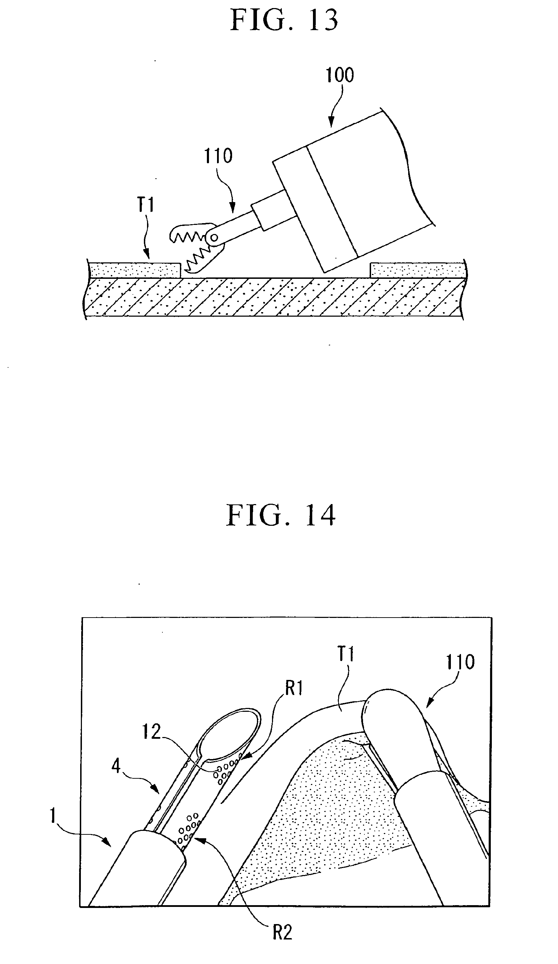

Next, as shown in FIG. 12, the operator protrudes the distal end of the grasping forceps 110 from the distal end of the endoscope device 100. As shown in FIG. 13, a biological tissue T1 around the portion where the mucous membrane M1 is located is resected by the grasping forceps 110, and puncturing of the suture instrument 1 is prepared.

FIG. 14 shows an image taken by the endoscope device 100 when the biological tissue T1 is grasped by the grasping forceps 110. Since it is possible to confirm the first indicator region R1 and the second indicator region R2 formed by the dimples 12 on the outer surface of the needle 4 of the suture instrument 1, the operator can easily determine the protrusion amount of the needle 4. Note that in the FIG. 14, the suture unit 103 is not shown for easier review.

The operator moves the distal end of the suture instrument 1 closer to the biological tissue T1 with the needle 4 protruded, and punctures and penetrates the needle 4.

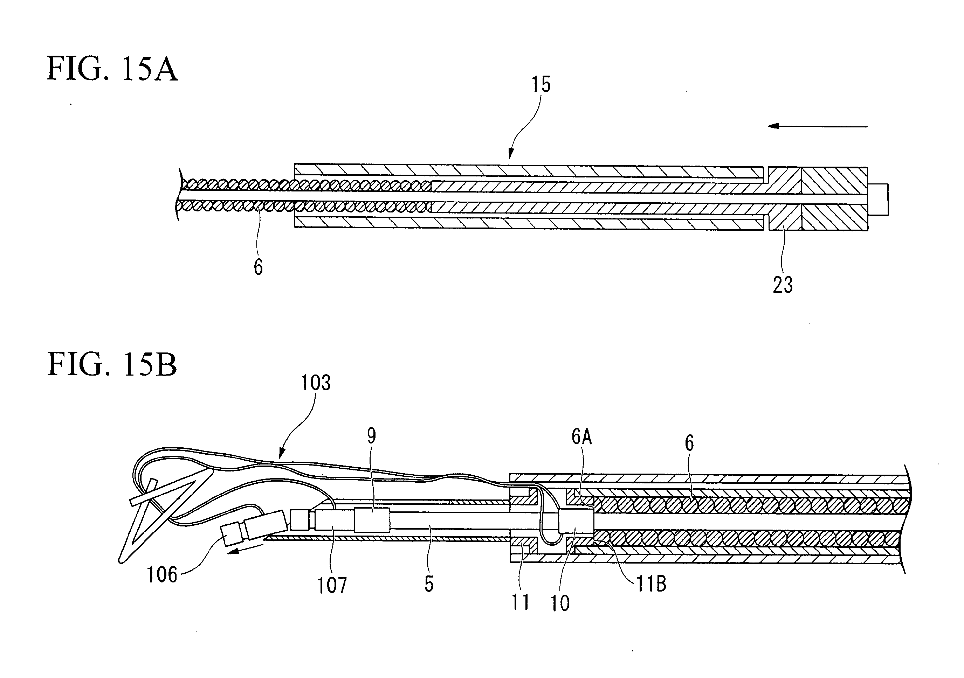

When the needle 4 is penetrated, as shown in FIG. 15A, the operator pushes forward the sheath operation member 23 of the distal end operation portion 15. Then the second sheath 6 slides forward. At this moment, as shown in FIG. 7B, since the distal end 6A of the second sheath 6 abuts the abutting member 10 of the wire 5, the wire 5 moves forward with the second sheath 6 by being pushed by the second sheath 6 with the constant relative position maintained.

The operator pushes the sheath operation member 23 until the distal end 6A of the second sheath 6 abuts the proximal end 11B of the connection pipe 11 and the movement of the second sheath 6 in a forward direction is restricted. Then as shown in FIG. 7B, the first anchor 106 and the second anchor 107 move forward by being pushed by the pushing member 9 at the distal end of the wire 5, and the first anchor 106 of the suture unit 103 is discharged outside of the needle 4. The operator can recognize the first anchor 106 being discharged by sensing that the second sheath 6 and the connection pipe 11 is abutted.

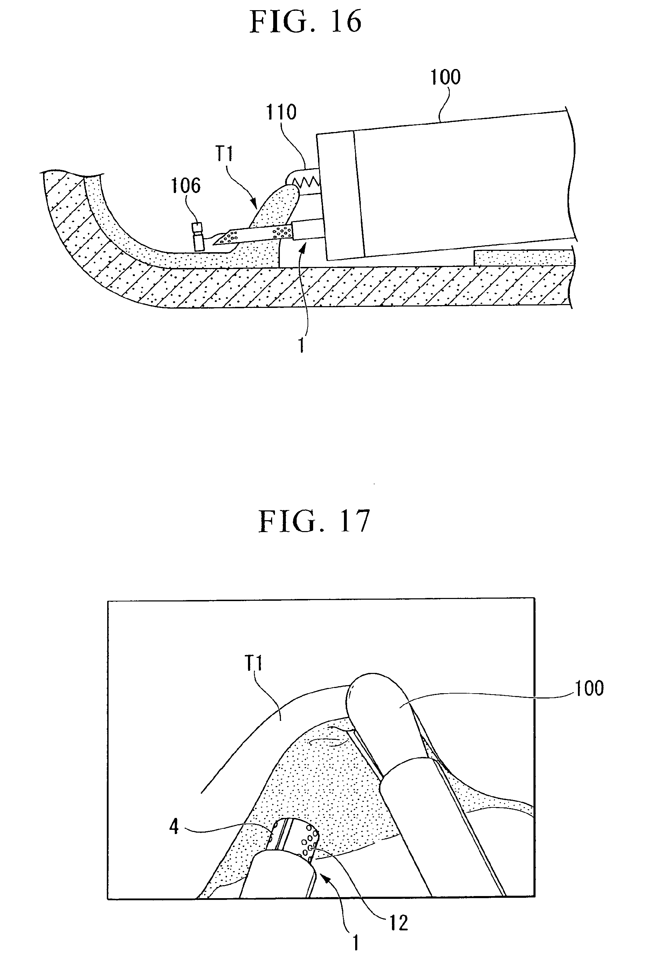

FIG 16 is a lateral view that shows a state when a first anchor 106 is discharged. The needle 4 penetrates the biological tissue T1 grasped by the grasping forceps 110. When the puncturing amount of the needle 4 becomes larger than necessary, it is difficult to adequately control the needle 4.

However, as shown in FIG. 17, in the needle 4 of the suture instrument 1, it is possible to confirm the dimples 12 on the image of the endoscope 100; it is possible to easily control the puncturing amount of the needle 4 with each of the indicator regions R1, R2, and the lines of the dimples 12 as an indicator at the time of the puncturing when discharging the anchors.

After discharging the first anchor 106, the operator withdraws the needle 4 from the biological tissue T1. At this time, the first anchor 106 is locked to the biological tissue T1. Next, the operator grasps a biological tissue T3 by the grasping forceps 110, which is facing the biological tissue T1 across the portion where the mucous membrane is resected, and punctures and penetrates the needle 4 of the suture instrument 1 with the same operations.

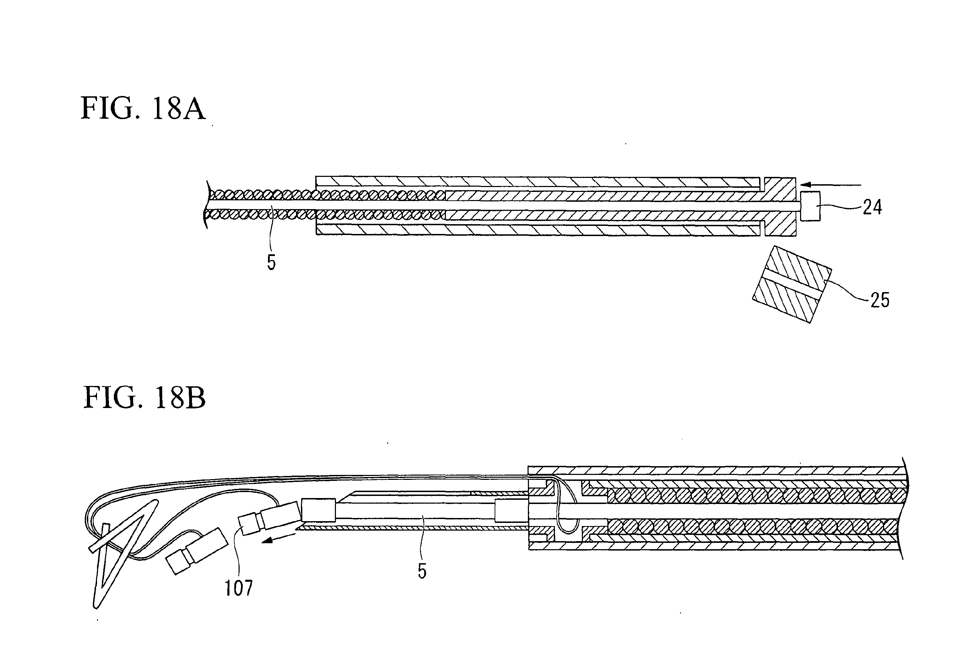

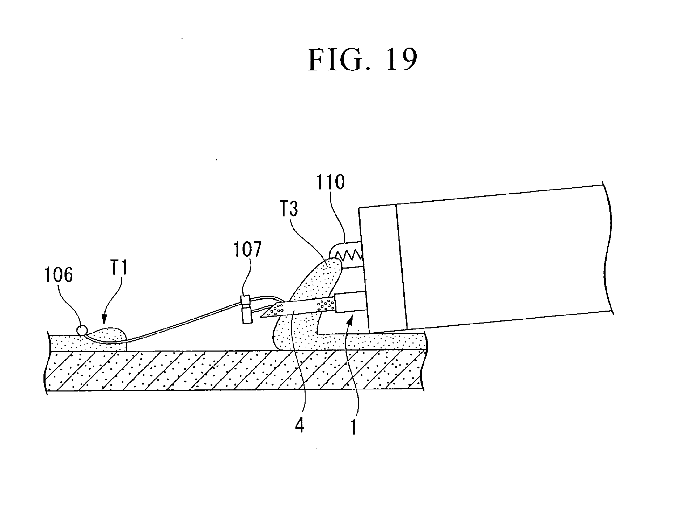

After the needle 4 has penetrated the biological tissue T3, as shown in FIG. 18A, the operator removes a wire stopper 25 and compresses the wire 5 forward by operating the wire operation knob 24. Then, as shown in FIGS. 18B, 19, the wire 5 moves forward further and the second anchor 107 is discharged to the outside of the needle 4. At this time also, since the operator can easily confirm the puncturing amount of the needle 4 by the dimples 12 on the image of the endoscope 100, it is possible to prevent a submucosal tissue or the like located forward from the biological tissue T3 from being damaged.

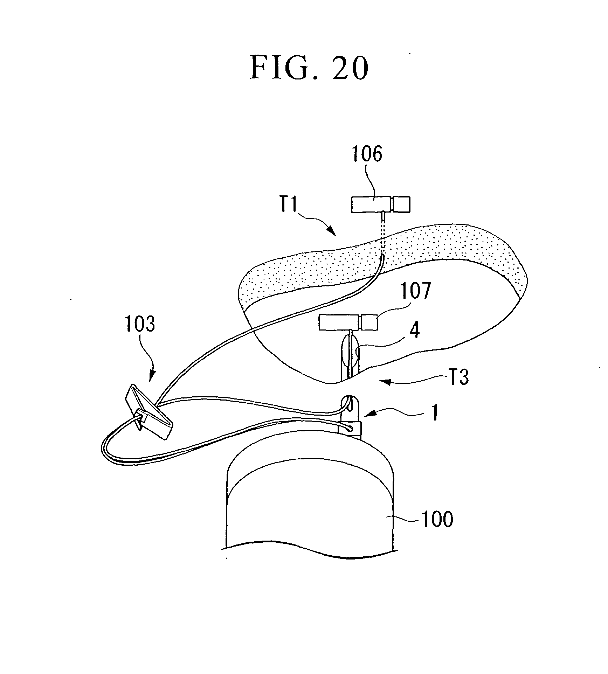

As shown in FIG. 20, after the second anchor 107 is discharged, the operator houses the grasping forceps 110 and performs a suturing operation by the suture unit 103. The operator withdraws the needle 4 from the biological tissue T3 and locks the second anchor 107 to the biological tissue T3.

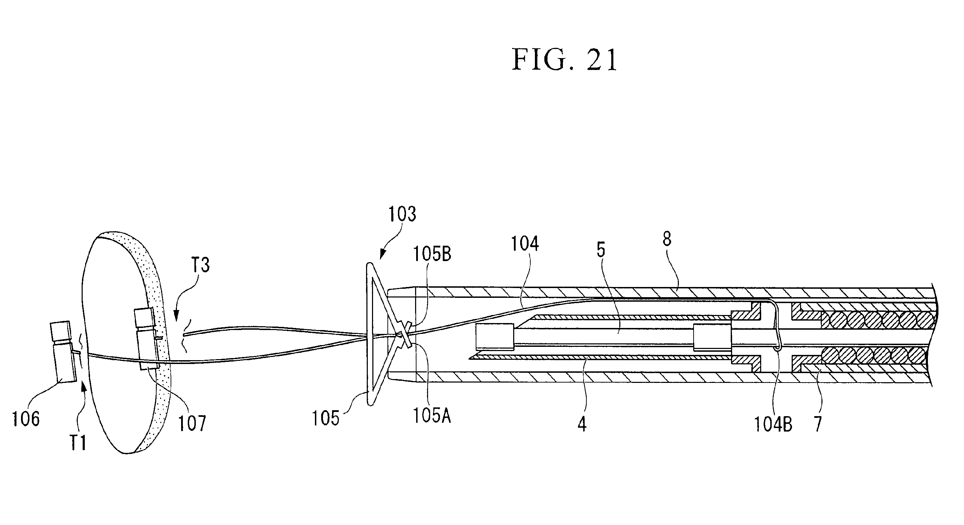

Next, the operator pulls the slider 19 to the side of the proximal end 13B of the main body 13, and houses the tube 7 and the needle 4 in the first sheath 8 as shown in FIG. 21. At this time, since the distal end operation portion 15 retracts with the slider 19, the wire retracts as well.

Then the suture thread 104 of the suture unit 103, which was placed around the wire 5, is housed into the first sheath 8, and the stopper 105 and the distal end of the first sheath 8 abut. When the operator further retracts the slider 19, only the suture thread 104 remains housed in the first sheath 8, with the stopper 105 and the first sheath 8 is abutted, and so the distances between the stopper 105 and each of the anchors 106, 107 shorten.

Since each of the anchors 106, 107 is locked to the biological tissues T1, T3 respectively, as the stopper moves closer to the each of the anchors 106, 107, the biological tissues T1, T3 are attracted to the side of the suture instrument 1 with each of the anchors 106, 107 and then adhere. In this manner, the portion where the mucous membrane is resected is sutured.

At this time, when the suture thread 104 moves to the side of the folding portion 104B and is housed in the sheath 8, the engagement between the edge portions 105A, 105B of the stopper 105 loosens. When the suture thread 104 is moved to the side of each of the anchors 106, 107, the suture thread 104 is not able to move in that direction since the edge portions 105A, 105B engage even more firmly due to a force acting on the suture thread 104. That is, since the stopper 105 moves only to the side of the anchors 106, 107 and does not move in the opposite direction, suturing of the portion where the mucous membrane is resected is not loosened or released.

After finishing the suturing, the operator removes the wire operation knob 24 and retracts the wire 5 relative to the tube 7. When the distal end of the wire 5 moves further backward than the connection pipe 11, the suture thread 104 is removed from the wire 5, the suture unit 103 is separated from the suture instrument 1, and the suturing of the portion where the mucous membrane is resected is finished. In this manner, a series of treatments are finished.

In accordance with the suture instrument 1 of the present embodiment, the plain region R3 is provided between the first indicator region R1 and the second indicator region R2 where the dimples 12 are disposed. Therefore, it is possible to use borders between each of the indicator regions R1, R2 and the plain region R3, that is, the anterior end of the first indicator region R1 on the side of the distal end or the front end of the second indicator region R2, as the indicators to easily confirm the puncturing amount of the needle 4.

Also, since the each of the indicator regions R1, R2, and R3 is formed to have a predetermined length in the axial line direction, it is possible to improve visibility to the operator compared to the examples that are only provided with scales or the like.

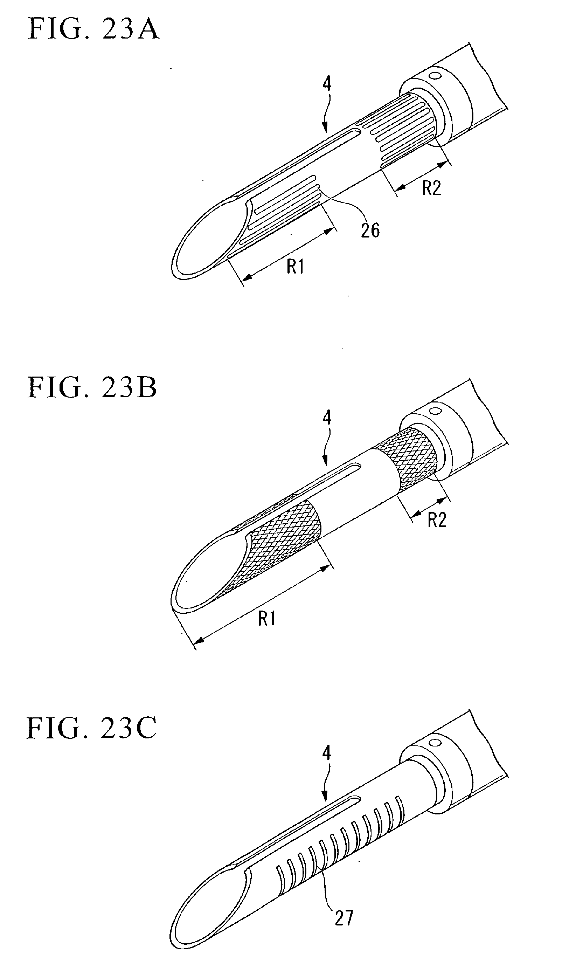

In the above embodiment, a case where the dimples 12 are provided on the surface of the needle 4 is described, but the method of providing indicator regions is not limited to this. For example, as shown in the alternative example in FIG. 23A, each of the indicator regions R1, R2 having miniature concave portions may be provided by providing a plurality of grooves 26, which are parallel to the axial line direction, on the outer surface of the needle 4. Or as shown in the alternative example in FIG. 23B, each of the indicator regions R1, R2 may be formed by providing a so-called SHIBO processing having miniature concavities and convexities. Or as shown in the alternative example in FIG. 23C, a plurality of grooves 27 along the circumferential direction is provided at predetermined intervals, and these grooves 27 may be used as indicator shapes. In this case, each of the grooves 27 functions as an independent indicator region.

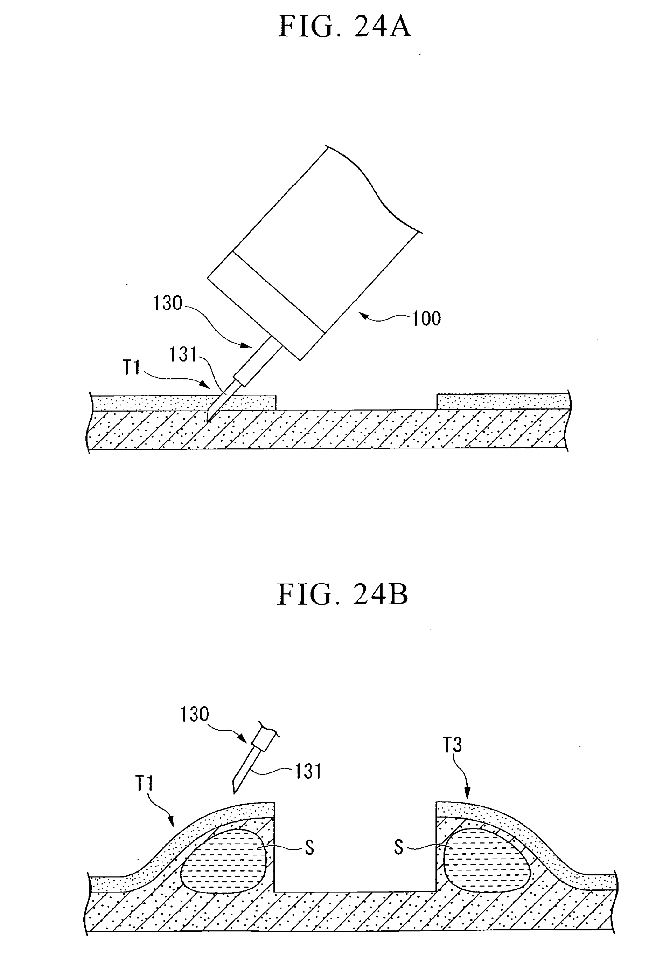

Next, an operation of the suture instrument 1 shall be explained when the suture instrument 1 is used in combination with a local injection. First, the operator inserts the endoscope device 100 in the same manner as described above and resects a biological tissue which is to be treated by the resecting instrument 120. Then, the resecting instrument 120 is withdrawn from the second channel 102, a local injection syringe 130 is inserted to the second channel 102 and is punctured to a biological tissue T1 around the portion where the mucous membrane is resected as shown in FIG. 24A. A saline solution S is injected to the inside of the biological tissue T1 from the injection needle 131 at the distal end of the local injection syringe 130 to swell the biological tissue T1 as shown in FIG. 24B. In the same manner, the saline solution S is injected to the biological tissue T3 that is facing the biological tissue T1 to swell the biological tissue T3.

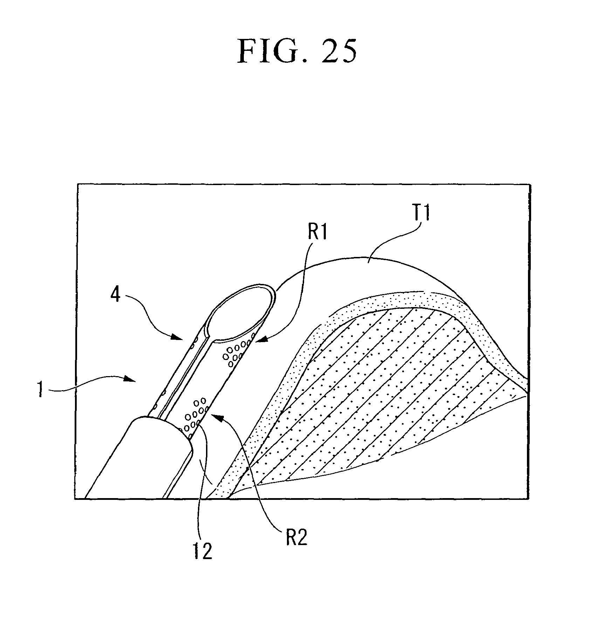

The operator confirms the distal end of the suture instrument 1 with the image of the endoscope device 100 shown in FIG. 25 with regard to the swelled biological tissue T1 and punctures the needle 4 to the inside of the biological tissue T1. At this moment, the operator confirms a substantial diameter of the swelled biological tissue T1 by using the dimples 12 and each of the indicator regions R1, R2 provided in the needle 4, and so it is possible to set the puncturing amount of the needle 4 based on the confirmed substantial diameter of the swelled biological tissue T1.

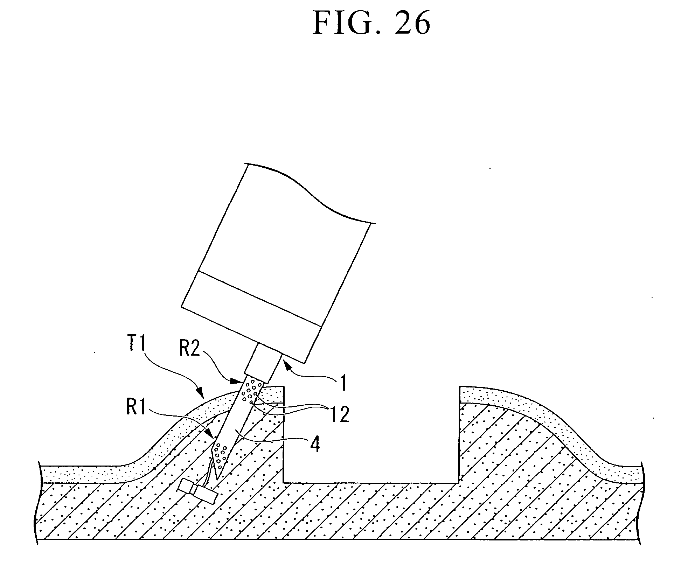

Next, the operator discharges the first anchor 106 to the inside of the biological tissue T1. FIG. 26 shows the first instrument 1 and the biological tissue T1 when a first anchor is discharged. Since a stomach wall extends outside by puncturing operations of the suture instrument 1 or the like, it is difficult to adequately control the needle 4 when the puncturing amount of the needle 4 is large. However, since indicator regions R1, R2 are provided on the needle 4 of the suture instrument 1 by the dimples 12, it is possible for the operator to easily confirm and adequately control the puncturing amount of the needle 4 by way of the image of the endoscope.

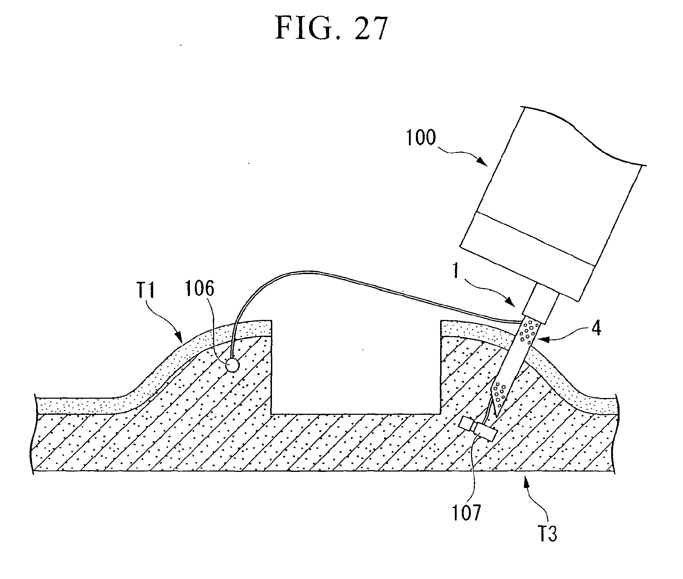

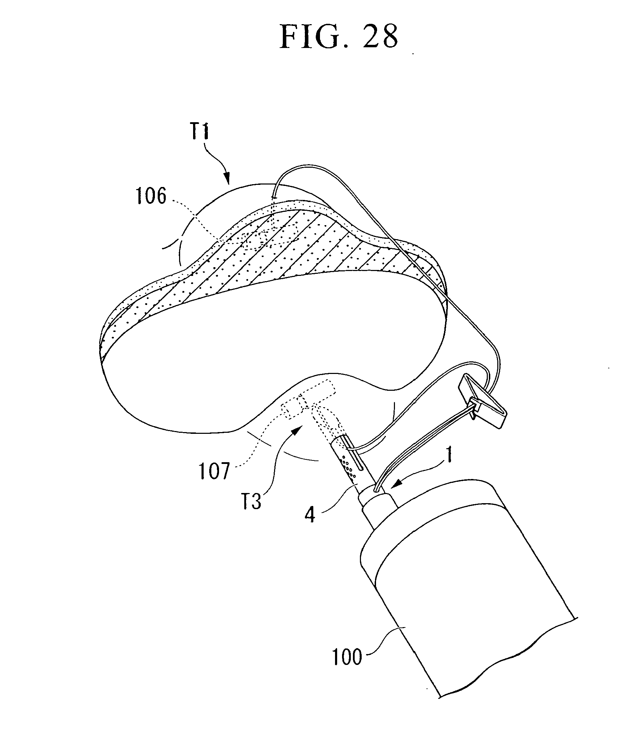

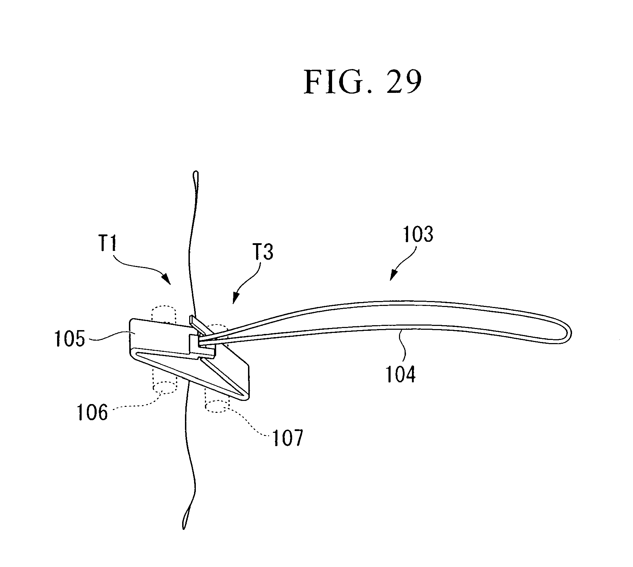

After discharging the first anchor 106, the operator punctures the needle to the biological tissue T3 by the same procedure, discharges the second anchor 107 to the inside of the biological tissue T3, and locks each of the anchors 106, 107 to the biological tissue, as shown in FIGS. 27, 28. Then as shown in FIG. 29, the biological tissues T1, T3 are attracted by using the suture unit 103 in the same manner as shown in the above-described case where the grasping forceps 120 is used and the portion where the mucous membrane is resected is sutured.

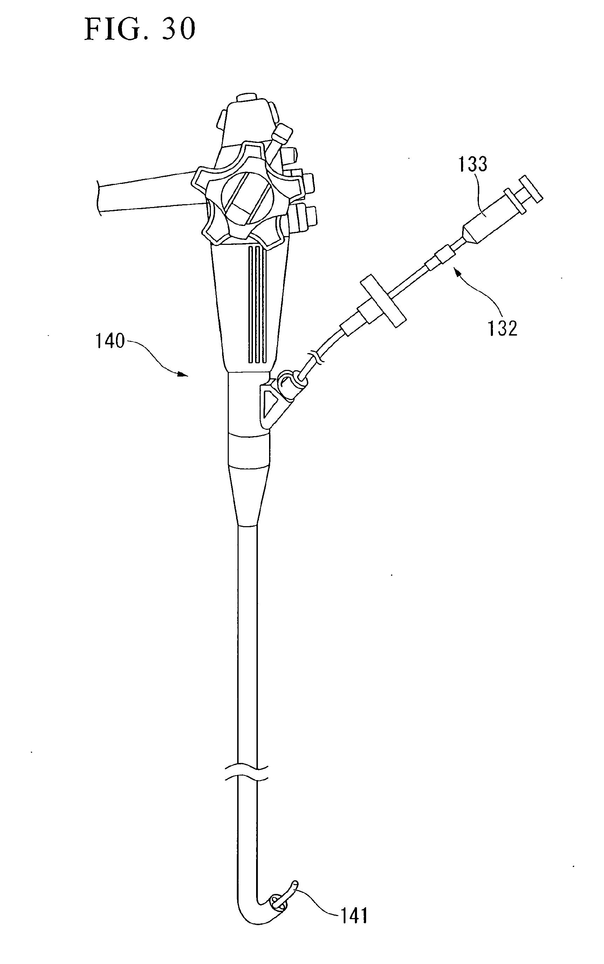

Next, the second embodiment of the present invention shall be described by referring to FIGS. 30 to 32. Note that, the same reference numerals shall be assigned to members that are the same as those used in the first embodiment, and descriptions thereof shall be omitted.

FIG. 30 shows a state in which a local injection syringe 132, which is an endoscope treatment instrument of the present embodiment, is used. The local injection syringe 132 has a known constitution which is able to inject a variety of medicines from an injection needle 134 (not shown) provided from a medicine inlet portion 133 at the side of the proximal portion to the distal end thereof. On the outer surface of the injection needle 134, a plurality of the dimples 12 is formed like the needle 4 of the suture instrument 1 as described later, and the first indicator region R1 and the second indicator region R2 are formed by the dimples 12.

An endoscope device 140, in which the local injection syringe 132 is inserted, is provided with an ultrasonic wave probe 141 which protrudes from the distal end, whereby in addition to a normal endoscopic image, it is possible to obtain an image around the probe 141 taken by ultrasound tomography.

As an example of operations when the local injection syringe 132 is used, a case in which anti-neoplastic agents are injected relative to a tumor located below a mucous membrane of the stomach by using the local injection syringe 132 shall be described.

First the operator inserts the endoscope device 140, in which the local injection syringe 132 is inserted in the channel thereof, to a body cavity of a patient. And the distal end of the endoscope device 140 is moved to the vicinity of the affected area to perform treatment inside of the stomach, and the injection needle 134 at the distal end of the local injection syringe 132 is protruded. Note that the local injection syringe 132 may be inserted in the channel after inserting the endoscope device 140 in the body cavity first.

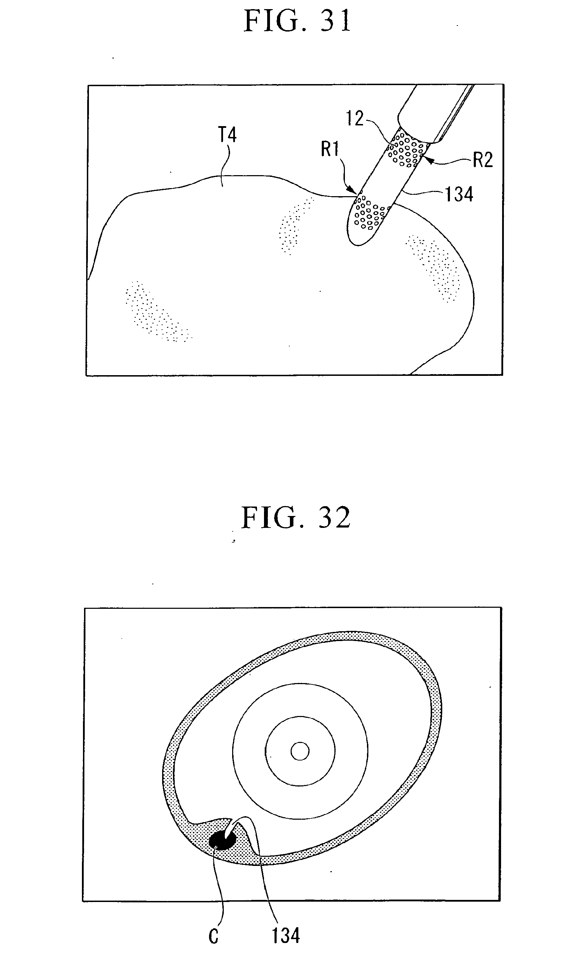

FIG 31 shows an image taken by the endoscope device 140. A biological tissue T4 to be treated is swelled due to a tumor located below. Since the dimples 12 are provided in the injection needle 134, like the suture instrument 1 of the first embodiment, the operator can utilize the dimples 12 and each of the indicator regions R1, R2, to confirm the diameter of the swelled biological tissue T4, and determine the puncturing amount of the injection needle 134.

After puncturing the injection needle 134, if necessary, the operator switches the image to the image taken by the ultrasound tomography using the ultrasonic wave probe 141. FIG. 32 shows an image taken by ultrasound tomography using the ultrasonic wave probe 141. Note that FIG. 32 shows the image taken by ultrasound tomography schematically and does not show the real image.

In the ultrasonic wave images, the dimples 12 provided in the injection needle 134 reflect diffusely the ultrasonic waves transmitted from the ultrasonic wave probe 141. Since the first indicator region R1 and the second indicator region R2 where the dimples 12 are disposed are formed, a brightness of the injection needle 134 in the ultrasonic wave image becomes higher. Accordingly, the operator can easily and precisely confirm the distal end position of the injection needle 134 inside of the biological tissue T1.

The operator confirms that the injection needle 134 punctures the tumor C, which is seen with different brightness from the normal stomach wall, injects the antineoplastic agents from the medicine inlet portion 133, and performs a local administration of the antineoplastic agents relative to the tumor C.

In accordance with the local injection syringe 132 of the present embodiment, since the dimples 12, which reflect ultrasonic waves diffusely, are provided in the injection syringe 134, it is possible to easily confirm and check the puncturing amount or position in the biological tissue of the injection needle 134 by using the endoscopic images by ultrasonic waves. Accordingly, it is possible to prevent adjacent organs or the like from being damaged by the distal end of the injection needle 134; it is possible to reliably perform local administration to a target region.

Note that since each of the shapes of the grooves 26 or the like in the alternative examples shown in FIGS 23A to 23C reflects ultrasound waves diffusely, it is possible to employ them as the injection needle 134. Here, since among these shapes, the dimples 12 reflect ultrasound waves diffusely the most, the dimples 12 are most preferable among these shapes.

In the present embodiment, an example in which antineoplastic agents are locally administered by using the local injection syringe 132 is explained, but objects to be treated by the local injection syringe are not limited to this example. For example, the local injection of a saline solution, when the suture instrument 1 of the first embodiment is used, may be performed by using the local injection syringe 132 of the present embodiment. Also, an injection syringe with a similar constitution may be punctured to the biological tissue, and may recover a part of the biological tissue in the inner cavity of the injection syringe for inspection.

Also, an object to be observed by the ultrasonic wave image is not limited to the local injection syringe 132, but the needle 4 of the suture instrument 1 can be checked by the ultrasonic wave image and the anchor may be discharged, for example, by confirming the position or the like of the needle 4 inside of the biological tissue.

Preferred embodiments of the present invention are explained above, but the present invention is not limited to these embodiments. Additions, omissions, replacement, and other modifications in the constitution can be made without departing from the spirit or scope of the present invention.

For example, in each of the embodiments, an example in which the plain region R3 is provided between the first indicator region R1 and the second indicator region R2. Instead of this example, the endoscope treatment instrument of the present invention may be constituted such that only a single indicator region having an indicator shape such as dimples or the like is provided. The endoscope treatment instrument of the present invention may be constituted such that the plain regions are provided on both sides in the axial line direction of the needle with respect to this indicator region. By constituting in this manner, it is possible to control by confirming a substantial puncturing amount of the needle by making the front end and rear end of this indicator region as an indicator.

Other than this, the invention is not restricted by the above description, but by only the scope of the appended claims.

## Claims

1. An endoscope treatment instrument having a needle for puncturing a biological tissue inside of a body cavity comprising:
an indicator region provided on an outer surface of the needle including an indicator shape having miniature concave portions, and
a plain region without the indicator shape provided in connection with at least one side of the indicator region in an axial line direction of the needle.

2. The endoscope treatment instrument in accordance with Claim 1, further comprising a first indicator region and a second indicator region provided in the indicator region, wherein
the plain region is provided between the first indicator region and the second indicator region.

3. The endoscope treatment instrument in accordance with Claim 1, wherein
the miniature concave portions of the indicator region are formed to reflect diffusely an ultrasound wave transmitted from outside in a radial direction of the needle.

4. A method of puncturing into a biological tissue in a body cavity using the endoscope treatment instrument in accordance with Claim 1 comprising:
setting a puncturing amount of the needle by confirming the indicator region of the needle on an endoscopic image.

5. The method of puncturing into a biological tissue in a body cavity using the endoscope treatment instrument in accordance with Claim 3 comprising:
setting a puncturing amount of the needle by confirming the indicator region of the needle on an endoscopic image, and
confirming the distal end position of the needle on an ultrasonic wave image after puncturing the needle into the biological tissue in the body cavity.
